# EUROPEAN PATENT SPECIFICATION

(11) **EP 0 877 939 B1**
(45) Date of publication and mention of the grant of the patent: **12.06.2002**
(21) Application number: 96944954.5
(22) Date of filing: 13.12.1996
(51) Int. Cl.: G01N 33/564, C12Q 1/48

(54) **METHODS FOR THE DETECTION OF ALZHEIMER'S DISEASE**
METHODEN ZUR FESTSTELLUNG VON ALZHEIMERSCHER KRANKHEIT
METHODES POUR DIAGNOSTIQUER LA MALADIE D'ALZHEIMER

(30) Priority: 19.01.1996 US 10250 P
(43) Date of publication of application: 18.11.1998
(73) Proprietor: Research Corporation Technologies, Inc, Tucson, Arizona 85711-3335 (US)
(72) Inventor: KAY, Marguerite, M., B., Temple, TX 76503 (US)
(74) Representative: Kolb, Helga, Dr. Dipl.-Chem.
(86) International application number: US9620465
(87) International publication number: WO9726537

(56) References cited:
- KAY M M B : "Band 3 in aging and neurological disease." ANNALS OF THE NEW YORK ACADEMY OF SCIENCES, vol. 621, 1991, pages 179 -205, XP002906229
- KAY M M B ET AL: "Alteration in the membrane protein band 3 associated with accelerated erythrocyte aging." PROCEEDINGS OF THE NATIONAL ACADEMY OF SCIENCES, vol. 86, no. 15, August 1989 (1989-08), pages 5834-5838, XP002906230
- KAY M M B ET AL: "Membrane protein band 3 alteration associated with neurologic disease and tissue-reactive antibodies" EXPL. CLIN. IMMUNOGENET., vol. 7, 1990, pages 181-199, XP002906231

## Description

### FIELD OF THE INVENTION

The present invention is directed to methods of detection of Alzheimer's disease. In particular, the present invention provides antibodies to the band 3 protein capable of differentiating Alzheimer's disease tissue from normal tissue. In addition, the invention provides a method of detection of Alzheimer's disease comprising determining differential band 3 phosphorylation.

### BACKGROUND OF THE INVENTION

Band 3 is a ubiquitous anion exchange protein. It is present in nuclear, Golgi, mitochondrial and cell membranes, and in all tissues thus far examined. Band 3 proteins in nucleated cells participate in band 3 antibody-induced cell surface patching and capping. Band 3 maintains acid-base balance by mediating the exchange of anions, is the binding site for glycolytic enzymes, and is the major structural protein linking the plasma membrane to the cytoskeleton. Because of its central role in CO₂ respiration, band 3 is the most heavily used ion transport system in vertebrate animals.

The human erythrocyte band 3 protein is the prototype for all band 3 proteins. Based upon the translated sequence of cDNA, the human erythrocyte band 3 protein consists of 911 amino acids (Tanner, et al., 1988, Biochem. J. 256:703.) The N-terminal 397 amino acids are located within the cytoplasm of the cell. Species and tissue-specific variation in band 3 and the few known band 3 polymorphisms are located primarily in this "cytoplasmic" segment, which, by convention based on enzymatic cleavage studies refers to amino acids from 1 to about 400. Two crucial properties of the band 3 molecule, anion transport and antigenicity of the senescent cell antigen (SCA), map to the membrane associated "domain", which consists of the C-terminal -500 amino acids. Kay, et al. (1991) Trans. Med. Rev. 5:173-195.

SCA, a biomarker for aging and neurological disease, is generated by oxidation and the breakdown of band 3. SCA appears on old cells and acts as a specific signal for the termination of that cell by initiating the binding of IgG autoantibody and subsequent removal by phagocytes. Kay (1978) J. Supramol. Struct. 9:555-567. This appears to be a general physiologic process for removing senescent and damaged cells in mammals and other vertebrates. SCA has been found on all cells examined including neurons. Besides its central role in the removal of senescent and damaged cells, SCA is present in neurofibrillary tangles and senile plaques in Alzheimer's disease, is involved in the removal of neural cells and platelets, and the removal of erythrocytes (RBC) in clinical hemolytic anemias, sickle cell anemia, and malaria.

Band 3 performs the same structural and functional activities in adult brain as it does in erythrocytes and lymphocytes. Kay, et al. (1983) Proc. Natl. Acad. Sci. USA 80:6882-6886. Thus, it exhibits the same characteristics as that of band 3 found in erythrocytes and lymphocytes. Cover, et al. (1996) Life Sciences 58:655-664; Kay and Goodman (1996) Gerontology 11, in press. Band 3 ages as cells and tissues age. Initially, band 3 undergoes an, as yet, uncharacterized structural change. Following this structural change, band 3 undergoes cleavage in the transmembrane anion transport region, resulting in generation of SCA. The present inventor has developed antibodies against this "aged band 3" that bind to distinct regions of band 3 in old but not middle-aged or young cells. These regions, called "aging antigenic sites", on band 3 are located at residues 538-554 and 812-830 of the band 3 molecule. Aging of band 3 is associated with functional changes including decreased efficiency of anion transport, decreased glucose transport, increased degradation to smaller fragments, and increased binding of physiologic IgG autoantibodies in situ.

Saitoh, et al. (1992) Ann. NY Acad. Sci. 674:180 and Kay, et al. (1994) Ann. NY Acad. Sci. 719:419 have suggested that degradation of band 3 occurs in brain membranes from patients with Alzheimer's disease. By immunohistochemistry, an antibody to a band 3 synthetic peptide (pep-COOH, residues 812-827 as numbered by Tanner, et al.) stained more neurons in frontal cortex from Alzheimer's patients than from normal controls. Serum autoantibodies were increased in Alzheimer's disease patients to band 3 peptides 588-602 and 822-839 compared to controls. Kay (1991) Ann. NY Acad. Sci. 621:179 suggests that brain band 3 may be altered in Alzheimer's disease.

In accordance with the present invention, it has been surprisingly found that certain anti-band 3 antibodies can discriminate between normal red blood cells and red blood cells of Alzheimer's patients, thus allowing a convenient and reliable assay for the disease. Further, unexpected alterations in the phosphorylation state of band 3 in Alzheimer's patients provide another assay for the disease. Moreover, it has been discovered that posttranslational changes occur in RBC band 3 that parallel changes in brain band 3. These changes include decreased ³²P phosphate labeling of band 3 polypeptides in brain and RBC in vitro, increased degradation of band 3, alterations in band 3 recognized by polyclonal and monoclonal antibodies decreased anion and glucose transport by red blood cells and increased degradation of band 3 to smaller fragments.

### SUMMARY OF THE INVENTION

The present invention provides a method for the detection of Alzheimer's disease comprising: contacting an antibody that differentiates Alzheimer's band 3 from normal band 3 with a tissue sample from a patient suspected of having Alzheimer's disease under conditions whereby an antibody-antigen complex is formed; detecting said complex; and comparing the quantity of said complex with the quantity of complex formed under the same conditions with a control sample from a healthy individual. In a preferred embodiment the tissue sample is cerebral cortex, blood or a fraction thereof, red blood cells, white blood cells or red blood cell membranes.

The present invention further provides a method for the detection of Alzheimer's disease comprising: contacting an antibody that differentiates Alzheimer's band 3 from normal band 3 with a tissue sample from a patient suspected of having Alzheimer's disease under conditions whereby an antibody-antigen complex is formed and detecting said complex.

The present invention further provides a method for the detection of Alzheimer's disease comprising determining the amount of phosphorylation of band 3 protein or degradation products thereof in a first tissue sample of a patient suspected of having Alzheimer's disease, and comparing the quantity of said phosphorylation of band 3 protein or degradation products thereof to the quantity of phosphorylation of band 3 protein or degradation products thereof in a second tissue sample of a healthy control individual, wherein decreased phosphorylation in said first sample is indicative of Alzheimer's disease. In a preferred embodiment the tissue sample is cerebral cortex, blood or a fraction thereof, red blood cells, white blood cells or red blood cell membranes.

### BRIEF DESCRIPTION OF THE DRAWINGS

Figures 1A-1D are bar graphs depicting results of immunoblots of blood samples assayed with band 3 antibodies.

Figure 2 demonstrates decreased band 3 phosphorylation in old mice as compared to middle-aged mice.

Figure 3 demonstrates decreased band 3 phosphorylation in Alzheimer's patients as compared to control temporal cortex samples.

Figure 4 demonstrates binding of ³²Phosphate to Alzheimer's disease (AD) and control RBC band 3. band 3 protein bands were identified by antibody binding as detected with alkaline phosphatase. Phosphorylation was quantitated by ³²P binding.

Figure 4A demonstrates binding of antiphosphotyrosine antibodies to band 3 in frontal gyrus brain membranes or middle aged RBC membranes from AD and controls. A, Control frontal gyrus; B, AD frontal gyrus; C, AD middle-aged RBC membranes; D, Control middle-aged RBC membranes.

Figure 5 demonstrates binding of 980 antibody to AD but not control brain membranes. Lanes: A, E, G, H = control membranes; B, C, D, F = AD membranes. AB = Amido black stain for proteins; AR = autoradiograph.

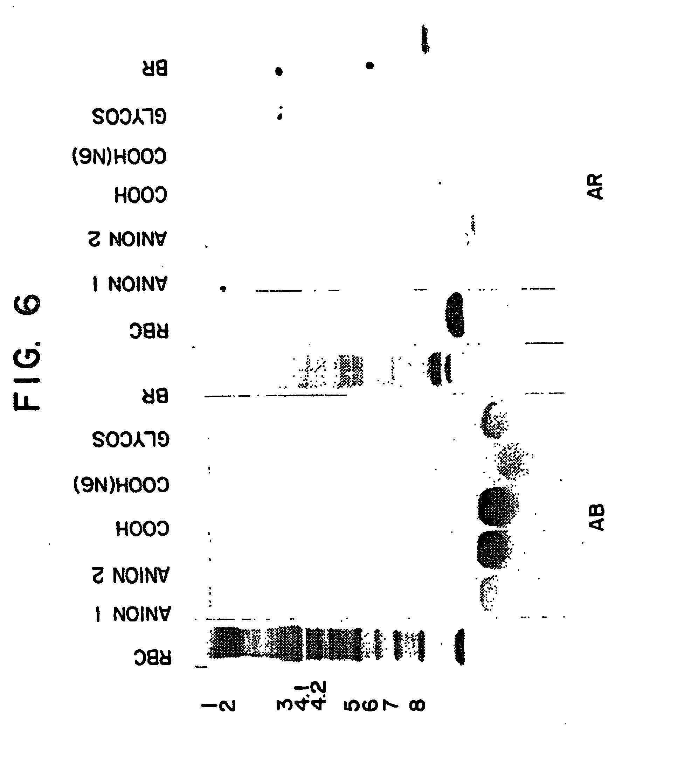

Figure 6 demonstrates that antibody 980 recognizes an internal band 3 anion transport segment, ANION 2, residues 588-602 (LRKFKNSSYFPGKLR) which is an aging vulnerable site for damage to band 3. ANION 2 (AN 2), residues 588-602 (LRKFKNSSYFPGKLR), with trace binding to COOH. ANION 2 appears to be intracellular because the potential N-glycosylation site at ASN-593 is not glycosylated. RBC, red blood cell membrane; ANION 1 (AN 1) = position 538-554: (SKLIKIFQDHPLQKTYN); COOH = 812-827: (LFKPPKYHPDVPYVKR); COOH-6, 813-818: (FKPPKY); Glycos, 630-648 (QKLSVPDGFKVSNSSARGW); BR, brain. Autoradiograph showing binding of antibodies to band 3 on the left and antibodies to aged band 3 on the right. AB = Amido black stain for proteins, AR = autoradiograph.

Figure 7A demonstrates antibodies AD-BD3 and peptide COOH differentiate between AD and control temporal cortex. Antibodies were tested against AD and control temporal cortex in immunodots. Other anti-band 3 peptide antibodies also distinguished AD from normal tissue.

Figures 7B and 7C demonstrate antibodies to band 3 peptides (AD-BD3 and pep-CYTO) differentiate between AD and control frontal cortex and middle-aged (MA) RBC in immunodots.

Figure 8 demonstrates examples of heterohybridoma antibodies to erythroid synthetic SCA that react more with AD tissue. Monoclonals from passages 1-3 were tested in immunodots. Antibody incubation was for 5h.

Figure 9 demonstrates examples of heterohybridoma antibodies to erythroid synthetic SCA that react more with control tissue. Monoclonals from passage 6 were tested in immunodots.

Figure 10 demonstrates antibodies to band 3 and synthetic peptides of band 3 distinguish AD from normal control brain in tissue sections. Electron microscopy was performed on etched sections of AD and control brain tissue reacted with antipeptide antibodies. Antibody binding was visualized with protein A attached to gold colloid. Antibodies to pep-COOH which recognizes band 3 degradation products (10A) and a glucose transporter channel protein (10B) reacted with AD but not control brain tissue. Antibodies did not react with control tissue (10C).

Figure 11 demonstrates heterohybridoma antibodies to erythroid SCA that differentiate between AD and control RBC.

Figure 12 demonstrates AD and control serum autoantibodies to synthetic peptides of band 3 and SCA quantitated by ELISA. Values shown are mean ± SEM.

### DETAILED DESCRIPTION OF THE INVENTION

The present invention provides methods for the detection of Alzheimer's disease. In particular, it has been discovered in accordance with the present invention that certain antibodies against band 3 or fragments thereof are capable of distinguishing between Alzheimer's and normal tissue samples. The antibodies are capable of distinguishing both blood and brain samples.

In a preferred embodiment, the antibody useful in the method of the present invention is an antibody against a species of band 3 known as "aged band 3". It is known in the art and described, for example, by Kay, et al. (1994) Ann. NY Acad. Sci. 719:419 that the transport membrane molecule band 3 undergoes an undefined change associated with aging which occurs prior to degradation of band 3 to SCA. This intact but altered band 3 is known as "aged band 3." Antibodies against aged band 3 can be identified by their ability to bind to band 3 in old but not middle-aged or young cells. Red blood cells can be easily separated into populations of different ages by well-known methods (Bennett, et al., 1981, Exp. Hematol. 9:297) to provide a source of cells to identify antibodies against aged band 3. A preferred antibody against aged band 3, antibody 980, is described by Kay, et al. (1989) Proc. Natl. Acad. Sci. USA 86:5834. Antibody 980 identifies aging changes in band 3 that occur prior to its formation of SCA. Kay (1991) Ann. NY Acad. Sci. 621:179-205. Another preferred antibody against aged band 3, antibody AD-BD3, is described by Kay, et al. (1990) Clin. Exper. Immunogenetics 7:181-199. AD-BD3 is made as an antiidiotype to senescent IgG (SC IgG), the autoantibody eluted from senescent erythrocytes. Antiidiotypic antibodies can be conventionally prepared by methods according to Kay (1985) Proc. Natl. Acad. Sci. USA 82:1731-1735. Pep-CYTO is an antibody against a cytoplasmic peptide of band 3, residues 122-144. Other antibodies against aged band 3 can be isolated as IgG autoantibodies from human serum or can be prepared by standard methods known to one of ordinary skill in the art. Both polyclonal and monoclonal antibodies are useful in the method of the present invention. Monoclonal antibodies, including rodent, rabbit, human, humanized, CDR-grafted and chimeric, are preferred. Human and humanized monoclonal antibodies against aged band 3 are particularly preferred. Stable, human antibody-secreting heterohybridomas were prepared by fusion of human B cells with a mouse myeloma cell line according to the methods of Thompson, et al. (1986) J. Immunol. 94:7-12.

It has been discovered by the present inventor that antibody 980 recognizes band 3 peptide residues 588-602. Residues 588-602 are also known as ANION 2. ANION 2 is an "aging vulnerable site". An "aging vulnerable site" as defined by the present invention is a band 3 region which contains a number of amino acids which are vulnerable to aging damage and initiate band 3 degradation. For example, Asparagine, at Position 593 deaminates and causes a concomitant change in band 3 configuration. ANION 2 is also vulnerable to serine proteases.

Accordingly, antibodies against peptide residues 588-602 are also contemplated in accordance with the present invention. Residues 588-602 have the sequence LRKFKNSSYFPGKLR.

In another preferred embodiment, the antibody useful in the method of the present invention is an antibody against a peptide having the residues 812-830 of band 3 as numbered by Tanner, et al. Residues 812 to 830 have the sequence
LFKPPKYHPDVPYVKRVKT.

In another preferred embodiment, the antibody useful in the method of the present invention is an antibody against a peptide having the residues 822-839 of band 3 (VPYVKRVKTWRMHLFTGI).

In another preferred embodiment, the antibody useful in the method of the present invention is an antibody against a peptide having the residues 538-554 of band 3 (SKLIKIFQDHPLQKTYN).

In another preferred embodiment, the antibody useful in the method of the present invention is an antibody against a peptide having the residues 853-870 of band 3.

In still another preferred embodiment, the antibody useful in the method of the present invention is an antibody against a peptide having the residues 122-144 of band 3 (DLQETSLAGVANQLLDRFIFEDQ).

In still another preferred embodiment, the antibody useful in the method of the present invention is an antibody against a peptide having the residues 812-827 of band 3 (LFKPPKYHPDVPYVKR).

In still another preferred embodiment, the antibody useful in the method of the present invention is an antibody against a peptide having the residues 526-541 of band 3 (FLISLIFIYETFSKLI).

In still another preferred embodiment, the antibody useful in the method of the present invention is an antibody against a peptide having the residues 549-566 of band 3 (LQKTYNYNVLMVPKPQGP).

In still another preferred embodiment, the antibody useful in the method of the present invention is an antibody against a peptide having the residues 561-578 of band 3 (PKPQGPLPNTALLSLVLM).

In still another preferred embodiment, the antibody useful in the method of the present invention is an antibody against a peptide having the residues 573-591 of band 3 (LSLVLMAGTFFFAMMLRKF).

In still another preferred embodiment, the antibody useful in the method of the present invention is an antibody against a peptide having the residues 597-614 of band 3 (FPGKLRRVIGDFGVPISI).

In still another preferred embodiment, the antibody useful in the method of the present invention is an antibody against a peptide having the residues 818-827 of band 3 (YHPDVPYVKR).

In still another preferred embodiment, the antibody useful in the method of the present invention is an antibody against a peptide having the residues 818-823 of band 3 (YHPDVP).

In still another preferred embodiment, the antibody useful in the method of the present invention is an antibody against a peptide having the residues 822-827 of band 3 (VPYVKR).

In still another preferred embodiment, the antibody useful in the method of the present invention is an antibody against a peptide having the residues 804-811 of band 3 (QLFDRILL).

In still another preferred embodiment, the antibody useful in the method of the present invention is an antibody against a peptide having the residues 828-834 of band 3 (VKTWRMH).

In still another preferred embodiment, the antibody useful in the method of the present invention is an antibody against a peptide having the residues 534-540 of band 3 (YETFSKL).

In still another preferred embodiment, the antibody useful in the method of the present invention is an antibody against a peptide having the residues 538-544 of band 3 (SKLIKIF).

In still another preferred embodiment, the antibody useful in the method of the present invention is an antibody against a peptide having the residues 543-549 of band 3 (IFQDHPL).

In still another preferred embodiment, the antibody useful in the method of the present invention is an antibody against a peptide having the residues 547-553 of band 3 (PLQKTYN).

Antibodies against e.g. peptides 122-144, 812-830, 812-827, 588-602, 822-839, 538-554 and 853-870 can be prepared by standard methods known in the art using the peptides made by standard methods of peptide synthesis. Polyclonal and monoclonal antibodies are useful in the present method. Monoclonal antibodies, including rodent, rabbit, human, humanized, CDR-grafted and chimeric antibodies are preferred and can be produced by methods well-known in the art. Antibody production is described, for example, by Burton, et al. (1994) Adv. Immunol. 57:191; Canerari, et al. (1993) Int. J. Biol. Markers 8:147; Zaccolo, et al. (1993) Int. J. Clin. Lab. Res. 23:192; Potter, et al. (1993) Intl. Rev. Immunol. 10:103; Kalsi, et al. (1992) Autoimmunity 13:249; and Harlow et al., eds. (1988) Antibodies, A Laboratory Manual, Cold Spring Harbor Laboratories, Cold Spring Harbor, New York.

The above-identified antibodies are capable of differentiating band 3 in tissue samples from Alzheimer's patients from band 3 in tissue samples of normal age-matched subjects. Suitable sources for tissue samples include cerebral cortex, blood, blood fractions, red blood cells, red blood cell membranes and white blood cells. Blood samples are particularly preferred.

In accordance with the present invention, an antibody capable of discriminating the Alzheimer's band 3 from normal band 3 is contacted with the tissue sample from a patient suspected of having Alzheimer's under conditions whereby an antibody-antigen complex is formed. In a corresponding control sample, the same antibody is contacted with the age-matched control tissue sample under the same conditions. The resulting antibody-antigen complexes are then compared. A statistically significant increase in antibody reactivity in the sample of the patient suspected of having Alzheimer's disease relative to the control sample is indicative of the presence of the disease. Furthermore, if no antibody reactivity is observed in the control sample, then positive reactivity in the sample of the patient suspected of having Alzheimer's disease is indicative of the presence of the disease.

By "statistically significant" is meant a 10-60% difference in antibody reactivity in a sample from a patient suspected of having Alzheimer's disease relative to a control sample and/or positive reactivity in the sample from a patent having Alzheimer's disease and no antibody reactivity in the control sample.

In accordance with the present invention, an antibody capable of discriminating the Alzheimer's band 3 from normal band 3 is contacted with the tissue sample from a patient suspected of having Alzheimer's disease under conditions whereby an antibody-antigen complex is formed. Conventional methods known to the skilled artisan are then employed to detect the formation of an antigen-antibody complex.

Standard immunoassay methods known to one of . ordinary skill in the art can be used for antibody binding and detection and quantitation in accordance with the present method. Suitable immunoassays include solid phase and solution assays, radioimmunoassays, western blots, enzyme-linked immunosorbent assays, immunofluorescent assays, chemiluminescent assays, and bioluminescent assays. Assays, conditions, and detection methods are well-known to one of ordinary skill in the art.

The present invention further provides a method for the detection of Alzheimer's disease comprising determining the amount of phosphorylation of band 3 protein or degradation products thereof in a first tissue sample of a patient suspected of having Alzheimer's disease, and comparing the quantity of said phosphorylation of band 3 protein or degradation products thereof to the quantity of phosphorylation of band 3 protein or degradation products thereof in a second tissue sample of a healthy control individual, wherein statistically significant decreased phosphorylation in said first sample relative to the control sample is indicative of Alzheimer's disease. Furthermore, if no phosphorylation is observed in the control sample, then positive reactivity in the sample of the patient suspected of having Alzheimer's disease is indicative of the presence of the disease.

In a preferred embodiment the tissue sample is cerebral cortex, blood or a fraction thereof, red blood cells or red blood cell membranes.

It has been discovered in accordance with the present invention that band 3 and its fragments exhibit decreased phosphorylation in Alzheimer's disease.

Detection and quantitation of phosphorylation of band 3 and its fragments can be assessed by methods well known to one of ordinary skill in the art. For example, phosphorylation can be detected by observing shifts in molecular weight on SDS-PAGE gels, or by assessing reactivity with antiphosphotyrosine antibodies. In a preferred embodiment, phosphorylation is detected by incubating a homogenized tissue sample with labeled phosphate, preferably in the form of γ³²P-ATP, under conditions whereby the phosphate in band 3 is exchanged with ³²P, and detecting and quantitating the ³²P that becomes incorporated into band 3 proteins. ³²P-labeled band 3 can be detected by immunoassay with anti-band 3 antibodies as described hereinabove. Detailed methods for detection of phosphorylation are provided at Example 2 hereinbelow. In accordance with the present method, a statistically significant decrease in phosphorylation of band 3 or its degradation products in a tissue sample from a patient suspected of having Alzheimer's relative to an age-matched control sample is indicative of Alzheimer's disease.

It has been discovered in accordance with the present invention that brain band 3 undergoes distinct changes in Alzheimer's disease such as differences in glucose and anion transport, and that these changes are accompanied by changes in the peripheral blood as shown hereinbelow in Example 7.

Besides band 3 proteins, it has also been discovered that there are other proteins that also age and can be used to differentiate between Alzheimer's disease tissue and normal tissue. An example of such a protein is the Glucose Transporter Protein. For example, Hepatic Glucose Transporter Protein (HEPG2) and Insulin Regulated Glucose Transporter Protein (IRGT) and the like. In addition, it has also been discovered that there are fragments or antigenic determinants of said Glucose Transporter Proteins that complex with the antibody that differentiate Alzheimer's tissue from normal tissue, e.g. HEPG2 residues (QGGASQSDKTPEELFHP) and IRGT residues (MPSGFQQIGSEDG). As another example, an IRGT antigenic determinant is represented by the sequence SQQLSGINAVFYYST. Thus, any of these proteins can be used in lieu of the band 3 proteins or fragments in the methodologies described hereinabove to differentiate between Alzheimer's tissue and normal tissue, in accordance with the present invention.

The following examples further illustrate the invention.

### EXAMPLE 1

Antigenic changes in band 3 in aging and Alzheimer's diseased brain samples were detected by antibodies to band 3 and its peptide and breakdown products.

Polyclonal antibodies against the following proteins or peptides were obtained or generated in rabbits by standard methods:
Band 3 and breakdown products (band 3 Brk);
Cytoplasmic peptide of band 3 (Cyto);
residues 812-827 of the COOH terminus of band 3 (R812-827) ;
aged band 3 prior to generation of SCA (980);
channel peptide of the glucose transport protein (Gluc) residues 469-485 (QGGASQSDKTPEELFHP); and
residues 812-830 of the COOH terminus of band 3 (R812-827).

Middle-aged (7 months old) and old (12-13 months old) mice were sacrificed by cervical dislocation. The brain was removed under aseptic conditions and placed in ice-cold homogenization buffer (5 mM Hepes, 0.32 M sucrose, 5.0 mM imidazole, 5.0 mM benzamidine, 0.3 mM EGTA, 0.5 mM MgSO₄, 0.5 mM ZnSO₄, 5.0 mM glycerophosphate, 2.0 mM mercaptoethanol, 0.1 mg/ml Aprotinin (Sigma), 0.1 mg/ml Leupeptin (Sigma), 0.05 mg/ml Peptstatin A (Sigma), pH 8.0). The brain was homogenized for 5 min. with a "Tissue Tearor" (Brinkman) and subjected to differential centrifugation for 8 minutes, 4°C. at 5,000 rpm in a Sorvall RC-5B ultracentrifuge. The supernatant was decanted and placed on ice while the remaining pellet was further homogenized in homogenization buffer and centrifuged at the same settings. After repeating this 3 times, supernatants were then centrifuged for 30 min., 4°C., at 20,000 rpm. The supernatant was discarded and the pellet resuspended in homogenization buffer, 10 mM ATP, and 20% ultrapure glycerol, and stored at -70°C. for immunodot and phosphorylation assays.

The immunodot assay was used to compare band 3, altered band 3 and band 3 breakdown products in young and old mice and control and Alzheimer's disease patients. Controls in which either membranes or band 3 antibodies were omitted were determined, and were the same. The control value was subtracted from experimental points. All samples to be compared were run at the same time. Nitrocellulose membranes were spotted with brain homogenate protein at concentrations ranging from 2 ng to 18 µg per well. The membranes were then dried for 45 min. at room temperature and blocked for 60 min. in a Tris buffered saline (TBS)-Tween buffer containing 50 mM Trisma (pH 7.4), 0.1% Tween, and 6.0% nonfat dry milk (NFDM). Membranes were then washed 3 times, one minute each in TBS-Tween (Biorad) buffer, and incubated for 6 hours in polyclonal antibody. Each membrane was then washed 4 times for 4 minutes with TBS. After a final wash with TBS-Tween for 10 min., dishes were changed and each membrane was incubated in TBS-Tween with 6.0% NFDM. After one hour, membranes were incubated in 140 µl ¹²⁵I-Protein A (Amersham) and 100 ml TBS-Tween for two hours. Membranes were washed 4 times for 5 min. each in TBS-Tween and then 2 times for 30 min. Wet membranes were cut along the grid marks for cpm determination with a gamma counter (Beckman). All determinations were performed in triplicate.

Results of the immunoblot assays are presented in Table I.

**TABLE I**

| ANTIGENIC CHANGES IN AGING AND AD BRAIN DETECTED BY ANTIBODIES TO BAND 3 AND ITS BREAKDOWN PRODUCTS, AGED BAND 3, SYNTHETIC PEPTIDES OF BAND 3, SYNTHETIC SCA PEPTIDE, OR A GLUCOSE TRANSPORTER CHANNEL PEPTIDE (GLUC) | | | | |
|---|---|---|---|---|
| | **MOUSE** | **HUMAN FRONTAL CORTEX** | | |
| **ANTIBODY TO** | **MA** | **OLD** | **C** | **AD** |
| MOUSE BRAIN | 58498 | 62989 | ND | ND |
| | (1726) | (3685) | | |
| BAND 3 BRK | 45534 | 246006* | 28150 | 31555 |
| | (3006) | (40327) | (6296) | (2995) |
| CYTO | 27430 | 24537 | 4364 | 6705 |
| | (2149) | (2160) | (815) | (514) |
| R812-827 | 29875 | 33199 | 15027 | 6090 |
| | (1422) | (4811) | (8835) | (1210) |
| 980 | 27813 | 24483 | 1948 | 5181* |
| | (2046) | (2180) | (764) | (770) |
| GLUC | 7423 | 7338 | 2046 | 2266 |
| | (299) | (485) | (447) | (292) |
| 812-830 | 17578 | 8404+ | 2380 | 4406+ |
| | (2041) | (575) | (647) | (300) |
| MA, middle-aged; C, age-matched control; AD, Alzheimer's disease; results are expressed as cpm; *P≤0.0001, +P≤0.01, ND, not done. Antibodies to synthetic peptides of band 3 were reacted with brain tissue from young treated and untreated mice. CYTO, antibody against a cytoplasmic peptide of band 3, residues 122-144; BD3-BRK, antibody that recognizes band 3 and its breakdown products; 980, antibody against aged band 3 prior to generation of SCA (SCA); 812-827 and 812-830, antibodies against these peptide residues from the carboxyl terminus of the transport domain of band 3; GLUC, antibody against a channel peptide of the glucose transport protein. | | | | |

The data in Table I verify that an increase in band 3 breakdown is associated with age. The data further indicate that binding by antibodies 980 and 812-830 to Alzheimer's disease membranes is significantly increased relative to binding to normal brain membranes. Thus antibodies 980 and 812-830 distinguish Alzheimer's disease from normal brain tissue.

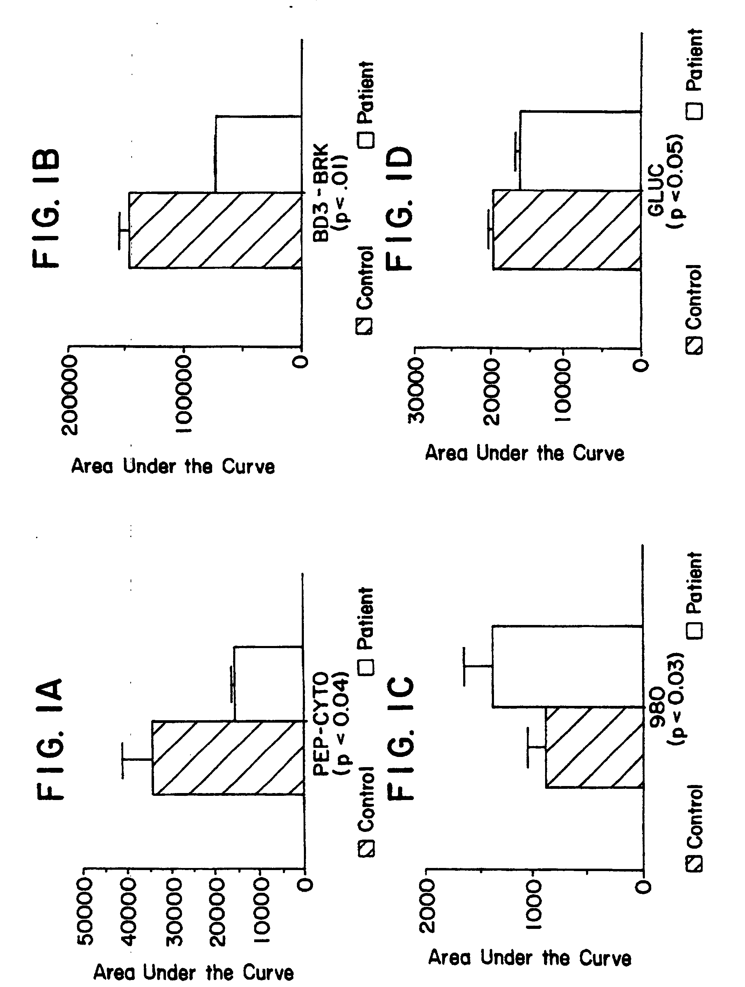

Immunodots using antibodies Cyto, band 3 Brk, 980 and Gluc were also performed on red blood cell membranes obtained from blood samples from Alzheimer's patients and normal age-matched controls. Results are graphed in Figures 1A-1D. As shown in Figure 1C, antibody 980 is also capable of distinguishing blood samples obtained from normal and Alzheimer's patients.

### EXAMPLE 2

The following example demonstrates band 3 phosphorylation changes in Alzheimer's disease versus normal age-matched controls. Phosphorylation was compared by quantitating the amount of ³²P binding to band 3 in membranes of lysed cells using SDS-PAGE.

The phosphorylation reaction was performed by mixing 100 µg brain homogenate in 50 µl homogenization buffer with 70 µl of 200 mM Tris-HCl, pH 7.6, containing Protein kinase A Inhibitor at a final conc of 100 µg/ml (Sigma), 200 mMMgSO₄, 2 mM ethylenedinitrilo-tetra acetic acid disodium salt (EDTA), 0.138 mM 2-mercaptoethanol, and γ³²P ATP, 0.15 mM (37 GBq/mmol) (ICN), and incubating for 12 minutes at 37°C. At the end of the phosphorylation reaction, samples were diluted with a 1M Tris-HCl buffer (pH 6.8) containing 40% glycerol and 25 mM EDTA. Fifty µg of each sample (75 µl) were loaded into a 6-23% sodium dodecyl sulfate polyacrylamide gel electrophoresis (SDS-PAGE) gel and allowed to run to within 2 cm from the bottom of the gel. Band 3 was identified by antibody/alkaline phosphatase staining of an immunoblot (western blot). A western blot was performed on polyvinylidine difluoride microporous membrane (PVDF:Millipore) membrane at 28 amps for 22 hours. Upon completion of transfer, the membrane was washed 3 times for 15 min each in a Tris Buffered Saline (TBS) containing 50 mM Trizma base, 3% fish gelatin, 0.1% Tween 20 then incubated for one hour in a polyclonal antibody to band 3 peptide residue 812-3 x 5 minutes in TBS and incubated for one hour in 50 µl goat anti-rabbit alkaline phosphatase (Zymed), 0.1% magnesium and zinc chloride, diluted in TBS. The membrane was again washed 3 times for 5 minutes each in 50 mM Tris pH 7.4), 0.1% Tween 20. Band 3 labeled antibodies were identified by incubating the membrane in 2.5% Diethanolamine, 0.3% 5-bromo-4-chloro-3-indolyl-beta-D-galactoside, 0.7% p-nitro blue tetrazolium chloride, until the color just began to develop. Radioactively phosphorylated proteins were identified by exposing the membrane in a lightning plus cassette at -70° C. for 24 hrs.

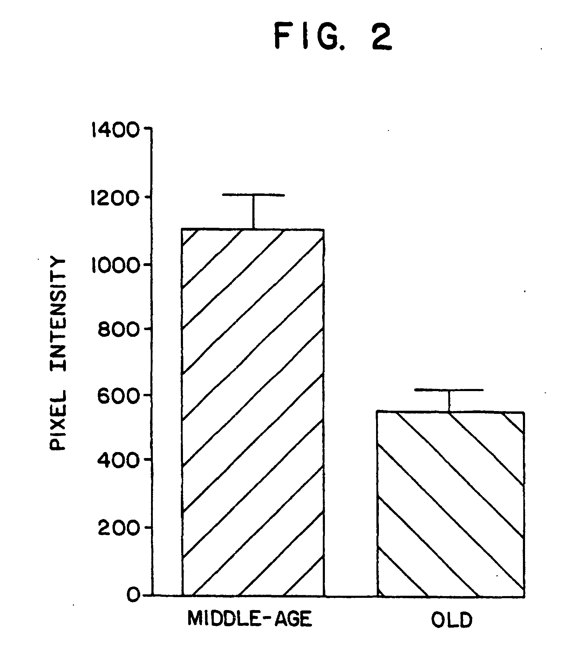

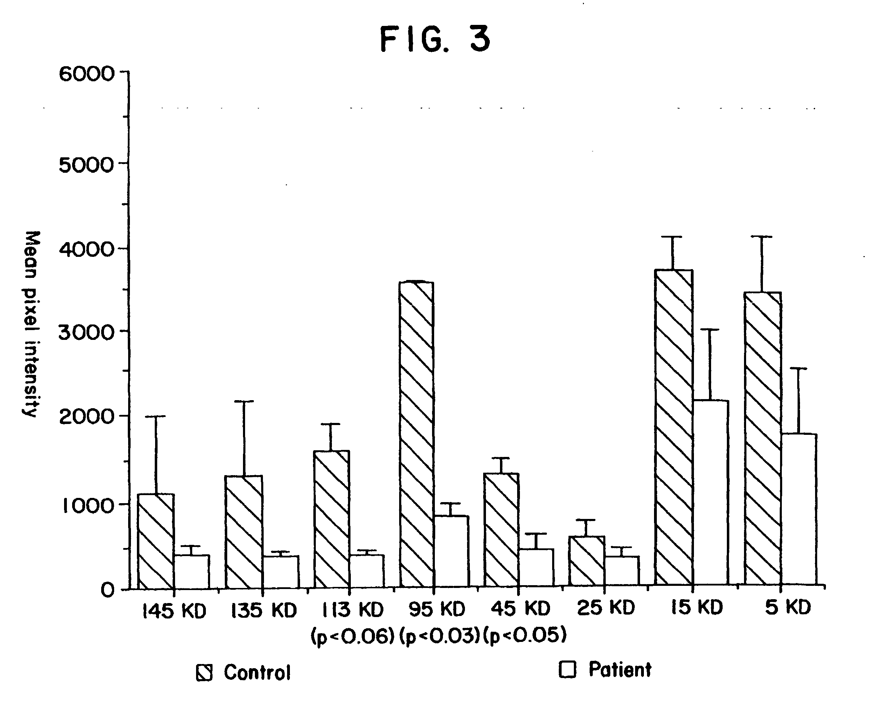

The results of this experiment indicated that phosphorylation was decreased in aged mice brains as compared to middle-aged and young brains (Fig. 2). Further, phosphorylation of band 3 and fragments was decreased in Alzheimer's disease versus age-matched controls (Fig. 3). The differential band 3 phosphorylation observed in Alzheimer's disease occurs at tyrosine residues.

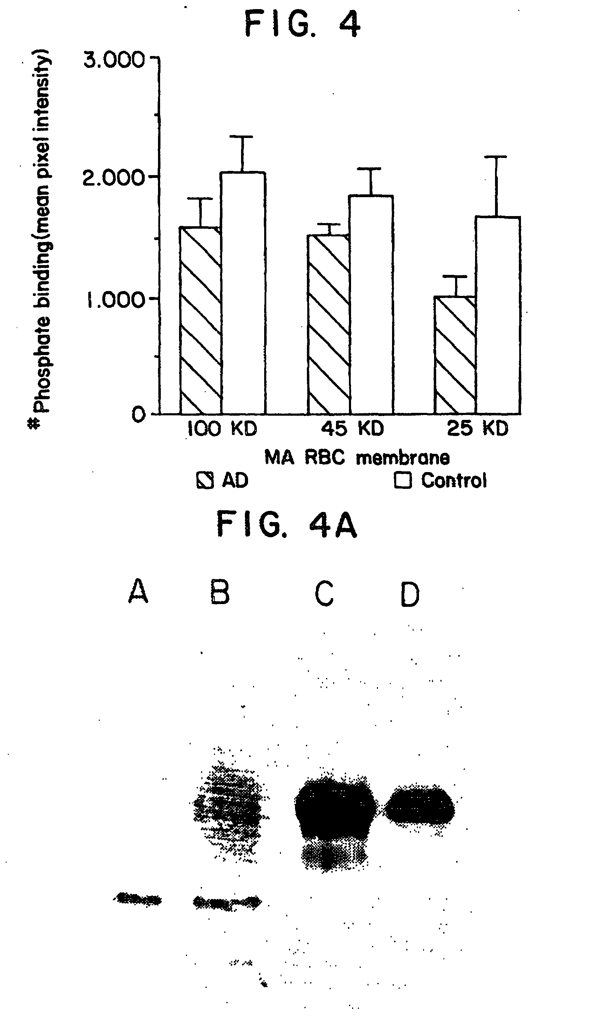

Phosphorylation of band 3 was also compared by quantitating the amount of ³²P binding to band 3 in the membranes of lysed red blood cells using SDS-PAGE as described for brain tissue. The results of this experiment indicated that phosphorylation was decreased in Alzheimer's disease red blood cells compared to age-matched controls (Figure 4).

As with the brain results, the decreased phosphorylation of band 3/binding of ³²P to red cells was due to increased phosphorylation in vivo (i.e. within the molecule), thus leaving fewer sites for ³²P labeling. Figure 4A shows binding of antiphosphotyrosine antibodies to band 3 in frontal gyrus brain membranes or middle-aged RBC membranes from AD and controls.

### EXAMPLE 3

The following example demonstrates in vitro studies with antibodies that distinguish between Alzheimer's disease and control brain tissue.

Brain tissue was obtained from Dr. J. Rogers, Sun City Research, Phoenix, Arizona and from Dr. Bosman, Department of Biochemistry, Faculty of Medicine, University of Nijmegan, The Netherlands.

The immunodot assay with antibodies described above to band 3 was performed as illustrated in the Examples above and as described by Cover, et al. (1996) Life Sci. 58:655-664. Nitrocellulose membranes were spotted with brain homogenetic protein at concentrations ranging from 2 ng and 18 µg as well. Membranes were dried, blocked and incubated for six hours with polyclonal band 3 antibody. Membranes were washed and incubated in 140 µl (0.14 µCi/ml) ¹²⁵I-protein A (specific activity, 37 MBq; 0.100 mCi/ml; Amersham) in 100 ml TGS-Tween for two hours. Washed membranes were cut along grid lines for cpm determination using a gamma counter (Beckman).

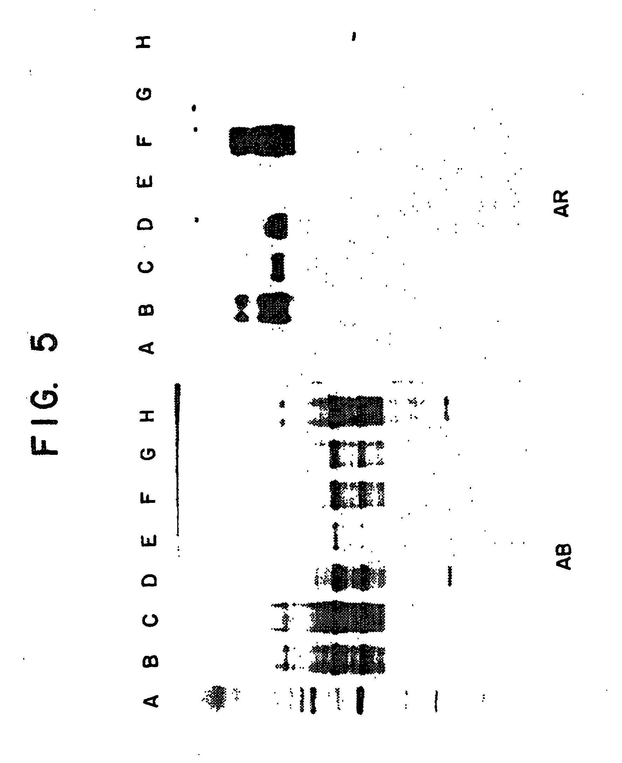

Results of the immunodot assays are presented in Figures 5 and 6. Figure 5 illustrates that antibody 980 distinguishes between Alzheimer's disease and normal brain membranes. Figure 6 illustrates the binding of antibodies to band 3 and to aged band 3. Antibody 980 recognizes a major anion transport segment of band 3 (ANION 2; Kay (1990) FASEB J. 5:109-115). ANION 2, residues 588-602, is an internal segment of band 3 to which antibody 980 binds strongly. Figure 6 also illustrates trace binding of antibody 980 to COOH, but not to ANION 1. Antibody 980 recognition of ANION 2 suggests that the band 3 change that precedes generation of SCA occurs in or near ANION 2, residues 588-602.

### EXAMPLE 4

The following example demonstrates that antibodies to band 3 differentiate between Alzheimer's disease and control temporal cortex and between Alzheimer's disease and control red blood cells.

Antibodies to synthetic peptides of band 3 differentiate band 3 proteins in brain membranes from normal individuals from those from patients with AD. One of these, an antibody to band 3 synthetic peptide pep-COOH detects a triplet in the 60,000-70,000 dalton range in brains from patients with AD but not from age-matched controls (see Saitoh, et al. (1992) Ann. NY Acad. Sci. 674:180-192). Antibodies to pep-COOH were used for immunohistochemical analysis of AD and normal frontal cortex. Pep-COOH antibodies were used because COOH is a component of senescent cell antigen. Pep-COOH antibodies only weakly stained neurons in normal cortex. Only a few large neurons were strongly reactive. In AD, the number of neurons labeling with antibodies to pep-COOH was increased by 260% (p ≤ 0.01). Because of a 50% neuronal loss in AD compared to controls (p ≤ 0.002), the proportion of neurons remaining that were pep-COOH-positive was increased by 540%. In AD, antibodies to pep-COOH labeled glial cells which were found around plaques. This suggests that band 3 exists in normal neurons in, predominately, an uncleaved state, and that degradation of band 3 occurs in AD thus allowing binding of antibodies to pep-COOH.

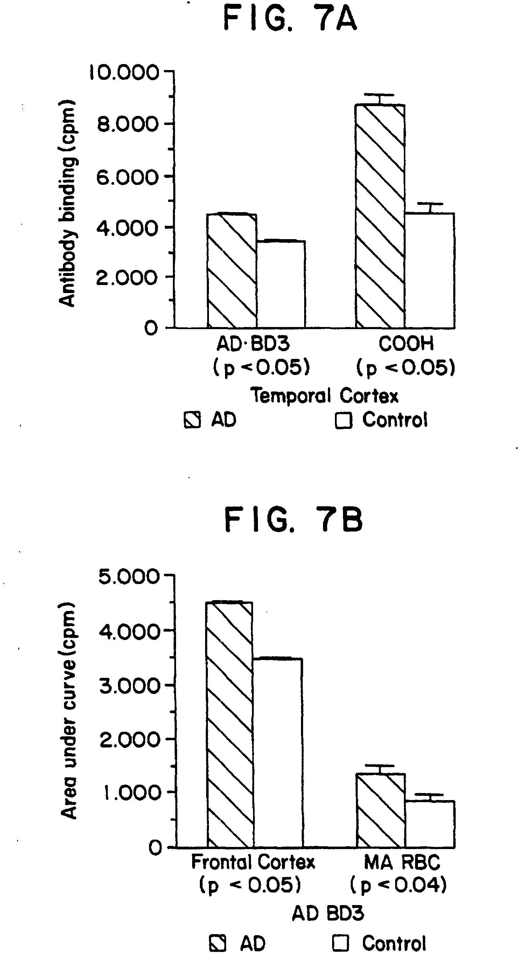

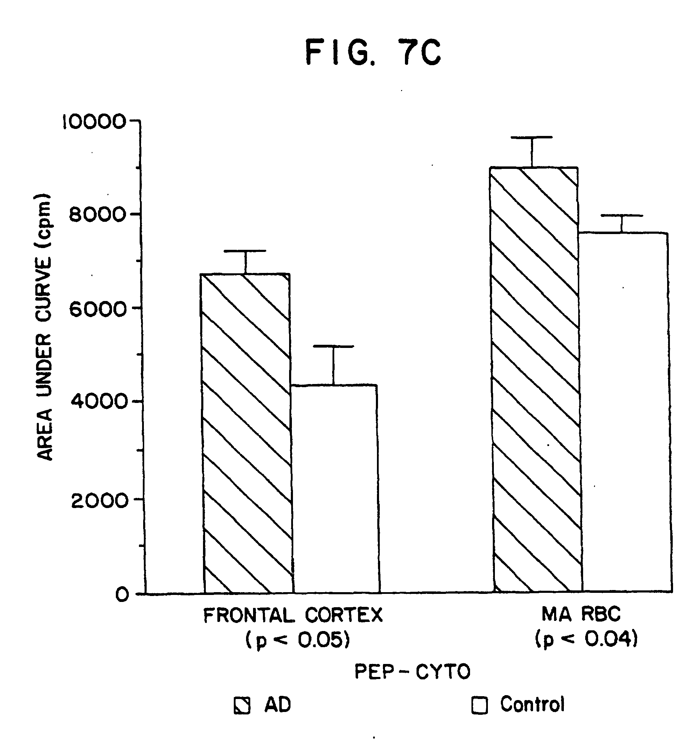

Figure 7A shows that there is a significant difference in binding to AD and control brains by a number of antibodies to band 3 including those to AD-BD3 and COOH which recognizes peptide breakdown products of band 3 and aged band 3 (p ≤ 0.001).

In addition to differentiating AD from normal brain, several anti-peptide antibodies differentiate AD from normal RBC. Sera were screened for antibodies to band 3 residues 538-554, 588-602, 812-827 using ELISA. These synthetic peptides duplicate key sequences of relevant portions of the band 3 molecule. Kudrycki & Shull (1989) J. Biol. Chem. 264:8185-8194. ELISA was performed essentially as described by Bennett & Kay (1981) Exp. Hematol. 9:297-307. Peroxidase conjugated goat anti-mouse immunoglobulin was used as the second antibody. Peptide antigens were dissolved in 0.2 m carbonate buffer at 1 mg/ml and 100 µg per well was used to coat the plates. After drying down overnight at 37°C, plates were washed three times with phosphate buffered saline containing .5% Tween 20 (PBST) and then quenched with blocking buffer, 0.01% gelatin (porcine skin) in PBST, for one hour on a rocker at room temperature. Plates were then washed three times with PBST and incubated for one hour with mouse serum serially diluted in blocking buffer with the initial dilution of 1:40 and final dilution of 1:5. Plates were washed and incubated for one hour with horseradish peroxidase conjugated goat anti-mouse IgG and IgM (heavy and light chain) (Caltag) at a 1:6400 dilution in blocking buffer. After washing with PBST, 0.03% 2-2' ANZO bis(3-Ethylbenzthiazoline-6-Sulfonic acid) diammonium salt in 0.1 M citrate buffer pH 4.0) with 0.01% H₂O₂ was added to the wells. The plates were read at 405 nm in a BioRad 3559 micro Plate Reader (BioRad Laboratories, Richmond CA) after 30 minutes.

The 'dot-blot'/immunodot assay was used to compare band 3, altered band 3, antipeptide antibodies, and band 3 breakdown products in red cells from individuals with AD and age-matched controls. There was a significant difference between red cell membranes from patients with AD and the spouse who was used as a control when reacted with the following antibody: AD-BD3 (p ≤ 0.04) (Figure 7A).

There were also significant differences between red cell membranes from patients with Alzheimer's disease and the spouse who was used as a control when reacted with the following antibodies: BD3-BRK (P≤0.01), a glucose channel protein (P≤0.05) and pep-CYTO. Thus, the same band 3 changes occur in erythrocytes and brain (Figures 7B-7C).

### EXAMPLE 5

The following example demonstrates that anti-SCA monoclonal antibodies can differentiate between Alzheimer's disease and control brain tissue.

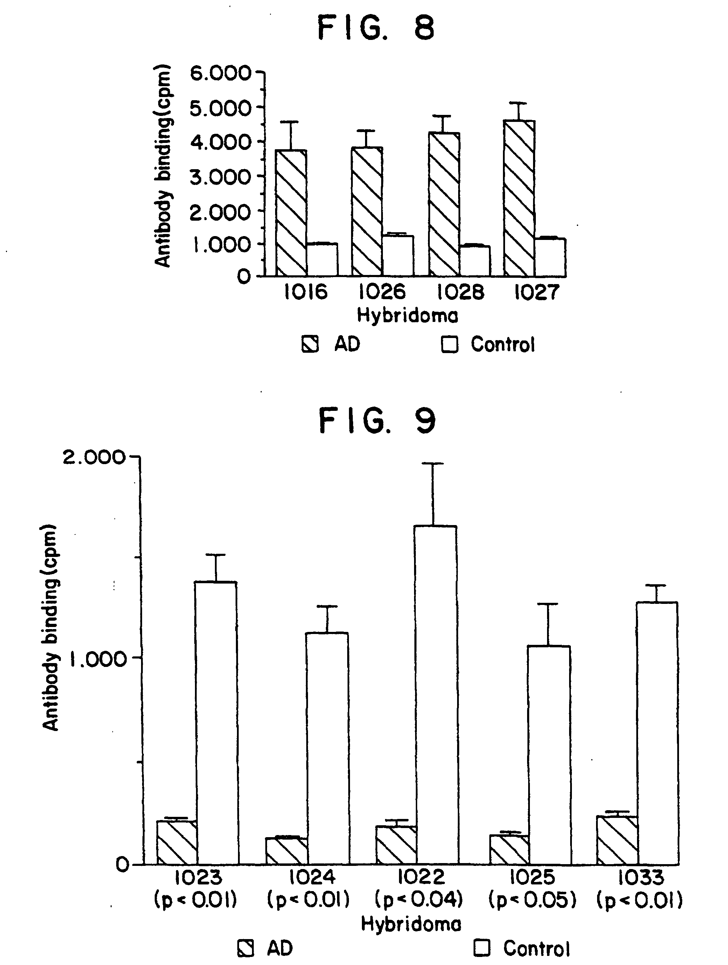

Naturally occurring B cells in peripheral blood of normal donors that produce antibody to SCA peptides ANION 1 and COOH were fused to the mouse P3X63-AG8.653 myeloma cell line to produce heterohybridomas. A number of heterohybridomas which screened against erythrocyte SCA, distinguished AD from control brain (Figures 8 and 9) and AD from control erythrocytes (see below). Some monoclonals exhibited increased binding to AD tissue while others exhibited increased binding to control tissue.

This suggests that (a) AD tissue lacks some antigenic determinants or combinations of determinants that normal tissue contains, and (b) AD tissue has acquired some antigenic determinants or combinations that normal tissue lacks. This indicates that normal human B cells produce antibodies to SCA. These antibodies require T-cell help suggesting that the age-related decline in immune function may contribute to AD. In addition, the fact that B cells from normal humans produce antibodies that distinguish AD band 3 from that of controls suggests that there are alterations in AD that can initiate, and others that can block, recognition by a healthy individual's immune system. This supports an immunological and/or autoimmune component to AD.

### EXAMPLE 6

This example demonstrates that antibodies to band 3 and synthetic peptides of band 3 distinguish Alzheimer's disease from normal control brain in tissue sections.

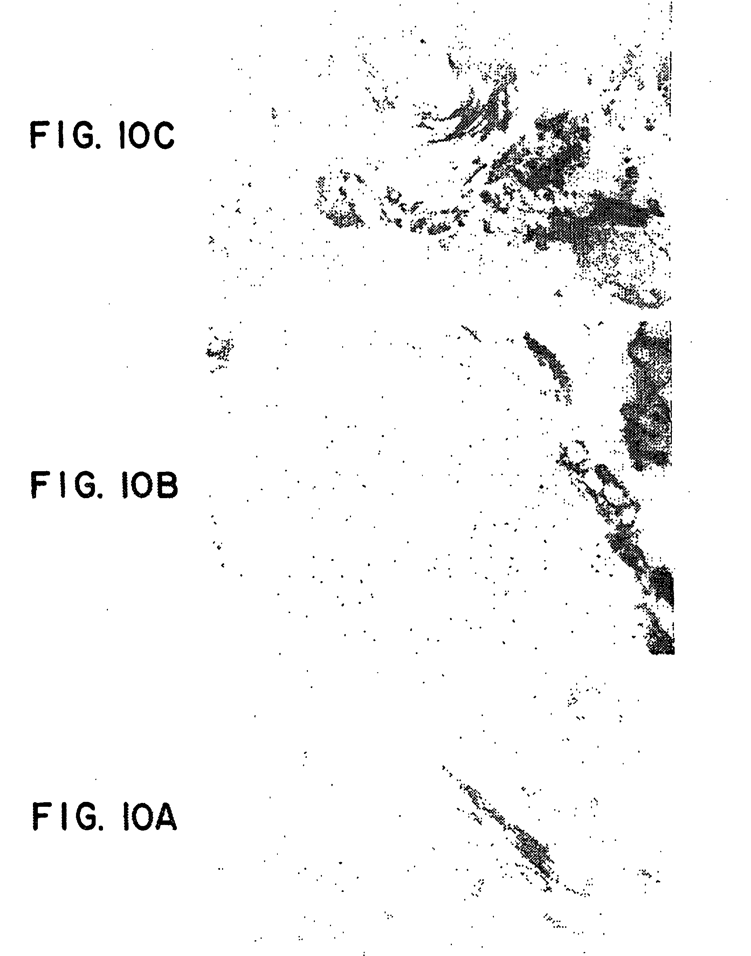

Immunoelectron microscopy was performed on etched sections of AD and control brain tissue reacted with antipeptide antibodies or preimmune rabbit serum. Antibody binding was visualized with protein A conjugated to gold colloid (Figure 10). Antibodies to pep-COOH and a glucose transporter channel protein reacted with AD but not control brain tissue. Normal rabbit serum did not react with AD or control tissue.

### EXAMPLE 7

This example demonstrates the differences observed in glucose and anion transport between 10 individuals with Alzheimer's disease and 10 age matched controls.

Anion and glucose transport differences can be detected in peripheral blood by methods well known to one of ordinary skill in the art. For example, sulfate uptake measurements were performed on middle-aged erythrocytes after separation on Percoll gradients as described by Cover, et al. (1996) Life Sciences 58:655-664. Erythrocytes were washed extensively in influx buffer (300 mM sucrose, 10 mM Trisma, pH 7.0) to remove external and intracellular chloride. After cells were resuspended in influx buffer, 2 x 10⁸ cells were aliquoted into borosilicated culture tubes for the assay to be performed in triplicate at five sulfate (substrate) concentrations. Prior to the addition of the appropriate substrate concentration, cells were incubated for 10 minutes at 37°C. At appropriate time intervals, 100 µl of substrate cocktail (400 mM K₂SO₄, 0.25 mCi Na₂³⁵SO₄) or one of its four subsequent serial dilutions (200, 100, 50 and 25 mM K₂SO₄), was added to the appropriate cell suspension tube so that the final substrate concentrations were 40, 20, 10, 5 and 2.5 mM, respectively. After incubating at 37°C for exactly 5 minutes, 0.5 ml of cell suspension was extracted and added to 9.0 ml ice-cod wash buffer (1.15 KCl, 10 mM MOPS, pH 7.4). Cells were then washed three times with wash buffer, centrifuged and all supernatants discarded. Cell pellets were deproteinized with 0.8 ml 10% trichloroacetic acid, centrifuged, and 600 µl of supernatant from each tube was added to 5.0 ml Opti-Fluor scintillation cocktail for determining β-emission. Sulfate uptake rates are expressed as nmol/10⁸ cells/min.

D-glucose uptake measurements were performed on middle-aged peripheral erythrocytes separated on Percoll gradients. Erythrocytes were washed extensively in 20 mM phosphate buffer (pH 7.4) to remove external and intracellular glucose. After cells were resuspended in phosphate buffer, 2 x 10⁸ cells were aliquoted into borosilicated culture tubes and put on ice for the assay to be performed in triplicate at five D-glucose (substrate) concentrations. At appropriate time intervals, 100 µl of substrate cocktail (20 mM D-glucose, 0.3 mCi D-³H-glucose) or one of its four subsequent serial dilutions (10, 5, 2.5 and 1.25 mM D-glucose) was added to the appropriate cell suspension tube so that the final substrate concentrations were 2, 1, 0.5, 0.25 and 0.125 mM, respectively. After incubating at 0°C for exactly 5 minutes, 0.5 ml of cell suspension was extracted and added to 9.0 ml ice-cold stop buffer (20 mM phosphate buffer, 0.1 mM phloretin, pH 7.4). Cells were then washed three times with stop buffer, centrifuged and all supernatants discarded. Cell pellets were deproteinized with 0.8 ml 10% trichloroacetic acid, centrifuged and 600 µl of supernatant from each tube was added to 5.0 ml Opti-Fluor scintillation cocktail for determination β-emission. D-glucose uptake rates are expressed as pmol/10⁸ cells/min.

Anion and glucose transport by AD patient's erythrocytes was consistently less than that of the paired control run in the same experiment. Because of the small sample size and the variability between individuals, the data was analyzed as the patient's transport relative to the control, which was usually the spouse. Differences in the V^{max} and Kₘ of glucose transport were statistically significant (p ≤ 0.04 and 0.03, respectively; Table II). Anion transport differences were also statistically significant for V^{max} (p ≤ 0.001) and Kₘ (p ≤ 0.04; Table II).

The rate of sulfate uptake decreased (i.e. anion transport decreased) with age in erythrocytes, brain and lymphocytes. Specifically, anion transport by splenic lymphocytes decreased by 24% with age (P≤0.005 for middle-aged compared to old mice; 30% for young compared to old mice, P<0.001). See Example 10.

Thus, RBC from individuals with Alzheimer's disease exhibited decreased efficiency of anion and glucose transport and a decrease in the number of anion and glucose binding sites (Kₘ) compared to their spouses and age-matched controls. Anion transport also decreased with age in lymphocytes.

**TABLE II**

| | Anion Transport¹ | | Glucose Transport² | |
|---|---|---|---|---|
| | V^{max} | Kₘ | V^{max} | Kₘ |
| Alzheimer's Controls | 16.5 ± 2.2 | 3.46 ± 0.16 | 214 ± 35 | 1.47 ± 0.41 |
| | 20.4 ± 1.0 | 3.16 ± 0.18 | 269 ± 43 | 1.81 ± 0.56 |
| | p ≤ 0.001³ | p ≤ 0.04 | p ≤ 0.04 | p ≤ 0.03 |

| | | | | |
|---|---|---|---|---|
| ¹ V^{max}, mmol/10⁸ cells/min.; Kₘ, mM. | | | | |
| ² V^{max}, pmol/10⁸ cells; Kₘ, mM. | | | | |
| ³ Statistical analysis (p value) was based on comparison . of the patient's transport to control transport for each experiment. The control was usually the spouse to compensate for environmental effects. | | | | |

### EXAMPLE 8

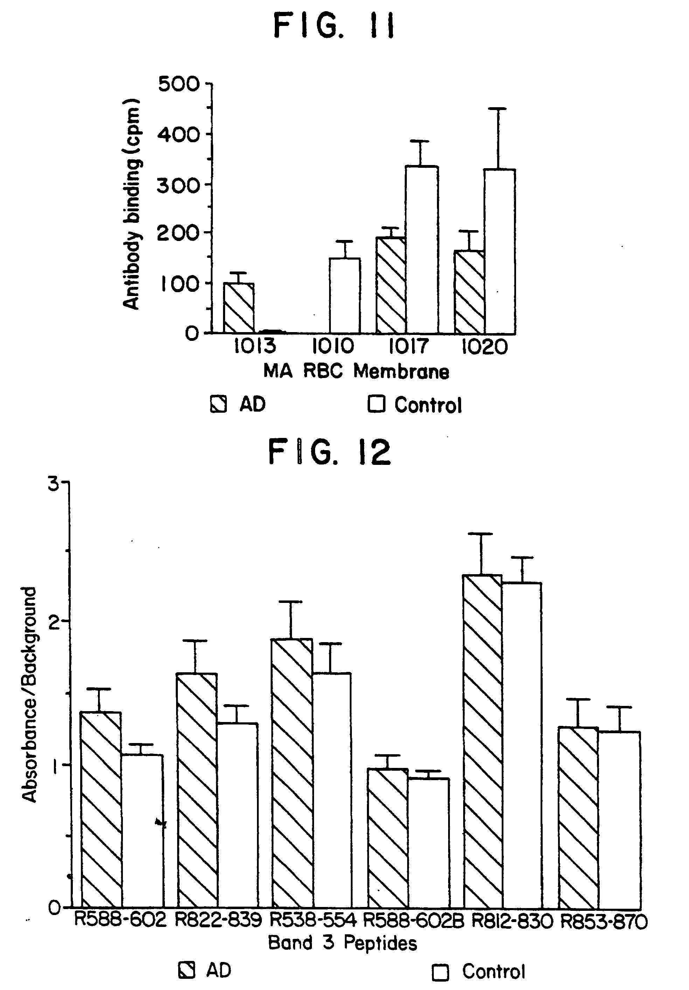

This example demonstrates that anti-SCA monoclonal antibodies can differentiate between Alzheimer's disease and control red blood cells. A number of the heterohybridomas described in Example 5 distinguished Alzheimer's disease from control erythrocytes (Figure 11). Some monoclonal antibodies reacted with Alzheimer's disease while others reacted with control red blood cells.

### EXAMPLE 9

This example demonstrates that serum autoantibodies to band 3 peptides 588-602 and 822-839 increased in Alzheimer's disease patients compared to controls.

Naturally occurring autoantibodies were quantitated to senescent cell antigen in human serum with ELISA during aging to determine whether these antibodies increased with AD. Naturally occurring autoantibodies may also be detected or quantitated by their reaction with known Alzheimer's disease tissues, e.g. brain.

Serum autoantibodies were increased in AD patients compared to controls to band 3 peptides 588-602 and 822-839 (two-tailed t test p ≤ 0.06; Figure 12). Significance is expected to increase as sample size increases. These band 3 residues lie in anion transport/binding regions. This is consistent with alteration of this region in AD since it is recognized as antigenically different by the patient's immune system. This is consistent with the observed decrease in anion transport (Example 7). Appearance of autoantibodies to the 588-602 region are consistent with data indicating that this is an "aging vulnerable site".

## Claims

1. A method for detecting Alzheimer's disease comprising:
(a) contacting a sample of blood or a fraction thereof, red blood cells, red blood cell membranes or white blood cells containing band 3 from a patient suspected of having Alzheimer's disease with an antibody that differentiates Alzheimer's band 3 from normal band 3 under conditions effective to form a complex between said antibody and band 3;
(b) detecting said complex;
(c) contacting a sample of blood or a fraction thereof, red blood cells, red blood cell membranes or white blood cells containing band 3 from a healthy individual with an antibody that differentiates Alzheimer's disease band 3 from normal band 3 and incubating for sufficient time and under conditions effective for a complex, if any, to form between said antibody and band 3; and
(d) comparing the amount of complex formed in step (b) with the complex formed in step (c), a statistically increased quantity of complex formed in step (b) relative to step (c) indicating the presence of Alzheimer's disease.

2. A method for detecting Alzheimer's disease comprising:
(a) contacting a sample of blood or a fraction thereof, red blood cells, red blood cell membranes or white blood cells containing band 3 from a patient suspected of having Alzheimer's disease with an antibody that differentiates Alzheimer's band 3 from normal band 3 under conditions effective to form a complex between said antibody and band 3; and
(b) detecting said complex.

3. The method of Claim 1 or 2 wherein said antibody is antibody 980.

4. The method of Claim 1 or 2 wherein said antibody is AD-BD3.

5. The method of Claim 1 or 2 wherein said antibody complexes with Peptides 812-827 of band 3.

6. The method of Claim 1 or 2 wherein said antibody complexes with Peptides 812-830 of band 3.

7. The method of Claim 1 or 2 wherein said antibody complexes with Peptides 822-839 of band 3.

8. The method of Claim 1 or 2 wherein said antibody complexes with Peptides 853-870 of band 3.

9. The method of Claim 1 or 2 wherein said antibody complexes with Peptides 538-554 of band 3.

10. The method of Claim 1 or 2 wherein said antibody complexes with Peptides 588-602 of band 3.

11. The method of Claim 1 or 2 wherein said antibody complexes with Peptides 469-485 of band 3.

12. The method of Claim 1 or 2 wherein said antibody Complexes with a mixture of Peptides 538-554 and 812-827 of band 3.

13. The method of Claim 1 or 2 wherein said antibody complexes with a mixture of Peptides 588-602 and 812-827 of band 3.

14. A method for detecting Alzheimer's disease comprising:
(a) determining the amount of phosphorylation of band 3 protein or degradation products thereof in a first tissue sample of a patient suspected of having Alzheimer's disease; and
(b) comprising the quantity of said phosphorylation of band 3 protein or degradation products thereof to the quantity of phosphorylation of band 3 protein or degradation products thereof in a second tissue sample of a healthy control individual; wherein statistically decreased phosphorylation in said first sample is indicative of Alzheimer's disease,

15. The method of Claim 14 wherein said tissue sample is brain tissue, blood or a fraction thereof, red blood cells, red blood cell membranes or white blood cells.

## Patentansprüche

1. Verfahren zum Nachweis von Alzheimer-Erkrankung, umfassend:
(a) In-Kontakt-Bringen einer Blutprobe oder einer Fraktion davon, roten Blutzellen, roten Blutzellmembranen oder weissen Blutzellen, enthaltend Bande 3 aus einem Patienten, der unter dem Verdacht steht, Alzheimer-Erkrankung zu haben, mit einem Antikörper, der die Bande 3 von Alzheimer von einer normalen Bande 3 unter Bedingungen unterscheidet, die dazu führen, einen Komplex zwischen diesem Antikörper und Bande 3 zu bilden;
(b) Nachweis des Komplexes;
(c) In-Kontakt-Bringen einer Blutprobe oder einer Fraktion davon, roten Blutzellen, roten Blutzellmembranen oder weissen Blutzellen, enthaltend Bande 3 von einem Gesunden, mit einem Antikörper, der Bande 3 der Alzheimer-Erkrankung von einer normalen Bande 3 unterscheidet, und Inkubieren für eine ausreichende Zeit, so dass sich ein Komplex zwischen dem Antikörper und Bande 3 bilden kann, wenn überhaupt; und
(d) Vergleichen der Menge an in Schritt (b) gebildetem Komplex mit dem in Schritt (c) gebildeten Komplex, eine statistisch erhöhte Menge an in Schritt (b) gebildetem Komplex relativ zu Schritt (c) zeigt das Vorhandensein von Alzheimer-Erkrankung an.

2. Verfahren zum Nachweis der Alzheimer-Erkrankung, umfassend
(a) In-Kontakt-Bringen einer Blutprobe oder einer Fraktion davon, roten Blutzellen, roten Blutzellmembranen oder weissen Blutzellen, enthaltend Bande 3 eines Patienten, der unter dem Verdacht steht, eine Alzheimer-Erkrankung zu haben, mit einem Antikörper der Bande 3 der Alzheimer-Erkrankung von einer normalen Bande 3 unter Bedingungen unterscheidet, die einen Komplex mit diesem Antikörper und Bande 3 ausbilden können; und
(b) Nachweis des Komplexes.

3. Verfahren gemäss Anspruch 1 oder 2, wobei der Antikörper der Antikörper 980 ist.

4. Verfahren gemäss Anspruch 1 oder 2, wobei der Antikörper der Antikörper AD-BD3 ist.

5. Verfahren gemäss Anspruch 1 oder 2, wobei der Antikörper mit den Peptiden 812-827 der Bande 3 komplexiert.

6. Verfahren gemäss Anspruch 1 oder 2, wobei der Antikörper mit den Peptiden 812-830 der Bande 3 komplexiert.

7. Verfahren gemäss Anspruch 1 oder 2, wobei der Antikörper mit den Peptiden 822-839 der Bande 3 komplexiert.

8. Verfahren gemäss Anspruch 1 oder 2, wobei der Antikörper mit den Peptiden 853-870 der Bande 3 komplexiert.

9. Verfahren gemäss Anspruch 1 oder 2, wobei der Antikörper mit den Peptiden 538-554 der Bande 3 komplexiert.

10. Verfahren gemäss Anspruch 1 oder 2, wobei der Antikörper mit den Peptiden 588-602 der Bande 3 komplexiert.

11. Verfahren gemäss Anspruch 1 oder 2, wobei der Antikörper mit den Peptiden 469-485 der Bande 3 komplexiert.

12. Verfahren gemäss Anspruch 1 oder 2, wobei der Antikörper mit einer Mischung von Peptiden 538-554 und 812-827 der Bande 3 komplexiert.

13. Verfahren gemäss Anspruch 1 oder 2, wobei der Antikörper mit einer Mischung der Peptide 588-602 und 812-827 der Bande 3 komplexiert.

14. Verfahren zum Nachweis der Alzheimer-Erkrankung, umfassend
(a) Bestimmung der Menge der Phosphorylierung des Proteins der Bande 3 oder der Abbauprodukte davon in einer ersten Gewebeprobe eines Patienten, der unter dem Verdacht steht, Alzheimer-Erkrankung zu haben,; und
(b) Vergleichen der Menge dieser Phosphorylierung des Proteins der Bande 3 oder der Abbauprodukte davon mit der Menge an Phosphorylierung des Proteins der Bande 3 oder der Abbauprodukte davon in einer zweiten Gewebeprobe eines Kontroll-Gesunden; wobei statistisch erniedrigte Phosphorylierung in der ersten Probe indikativ ist für eine Alzheimer-Erkrankung.

15. Verfahren gemäss Anspruch 14, wobei die Gewebeprobe Gehirngewebe, Blut oder eine Fraktion davon, rote Blutzellen, rote Blutzellmembranen oder weisse Blutzellen ist.

## Revendications

1. Procédé pour détecter la maladie d'Alzheimer, qui comprend :
(a) la mise en contact d'un échantillon de sang ou d'une fraction sanguine, de globules rouges, de membranes érythrocytaires ou de globules blancs contenant la bande 3 provenant d'un patient que l'on suspecte d'avoir la maladie d'Alzheimer, avec un anticorps qui fait la distinction entre la bande 3 d'un Alzheimer et la bande 3 normale dans des conditions appropriées à la formation d'un complexe entre ledit anticorps et la bande 3 ;
(b) la détection dudit complexe ;
(c) la mise en contact d'un échantillon de sang ou d'une fraction sanguine, de globules rouges, de membranes érythrocytaires ou de globules blancs contenant la bande 3 provenant d'un sujet sain, avec un anticorps qui fait la distinction entre la bande 3 de la maladie d'Alzheimer et la bande 3 normale, et l'incubation, pendant un laps de temps suffisant et dans des conditions permettant à un éventuel complexe de se former entre ledit anticorps et la bande 3 ; et
(d) la comparaison de la quantité du complexe formé dans l'étape (b) avec celle du complexe formé dans l'étape (c), une quantité statistiquement accrue du complexe formé dans l'étape (b) par rapport à l'étape (c) étant le signe de la présence d'une maladie d'Alzheimer.

2. Procédé pour détecter la maladie d'Alzheimer, qui comprend :
(a) la mise en contact d'un échantillon de sang ou d'une fraction sanguine, de globules rouges, de membranes érythrocytaires ou de globules blancs contenant la bande 3 provenant d'un patient que l'on suspecte de présenter la maladie d'Alzheimer, avec un anticorps qui fait la distinction entre la bande 3 de la maladie d'Alzheimer et la bande 3 normale dans des conditions permettant la formation d'un complexe entre ledit anticorps et la bande 3 ; et
(b) la détection dudit complexe.

3. Procédé selon la revendication 1 ou 2, dans lequel ledit anticorps est l'anticorps 980.

4. Procédé selon la revendication 1 ou 2, dans lequel ledit anticorps est l'AD-BD3.

5. Procédé selon la revendication 1 ou 2, dans lequel ledit anticorps se complexe avec les peptides 812-827 de la bande 3.

6. Procédé selon la revendication 1 ou 2, dans lequel ledit anticorps se complexe avec les peptides 812-830 de la bande 3.

7. Procédé selon la revendication 1 ou 2, dans lequel ledit anticorps se complexe avec les peptides 822-839 de la bande 3.

8. Procédé selon la revendication 1 ou 2, dans lequel ledit anticorps se complexe avec les peptides 853-870 de la bande 3.

9. Procédé selon la revendication 1 ou 2, dans lequel ledit anticorps se complexe avec les peptides 538-554 de la bande 3.

10. Procédé selon la revendication 1 ou 2, dans lequel ledit anticorps se complexe avec les peptides 588-602 de la bande 3.

11. Procédé selon la revendication 1 ou 2, dans lequel ledit anticorps se complexe avec les peptides 469-485 de la bande 3.

12. Procédé selon la revendication 1 ou 2, dans lequel ledit anticorps se complexe avec un mélange des peptides 538-554 et 812-827 de la bande 3.

13. Procédé selon la revendication 1 ou 2, dans lequel ledit anticorps se complexe avec un mélange des peptides 588-602 et 812-827 de la bande 3.

14. Procédé de détection de la maladie d'Alzheimer, qui comprend :
(a) la détermination d'une quantité de phosphorylation de la protéine bande 3 ou de produit de dégradation de cette dernière dans un premier échantillon de tissu d'un patient que l'on suspecte de présenter la maladie d'Alzheimer ; et
(b) la comparaison de la quantité de ladite phosphorylation de la protéine bande 3 ou des produits de dégradation de cette dernière et de la quantité de phosphorylation de la protéine bande 3 ou de produits de dégradation de cette dernière dans un deuxième échantillon de tissu d'un sujet témoin sain ; une phosphorylation ayant statistiquement subi une diminution dans ledit premier échantillon étant le signe d'une maladie d'Alzheimer.

15. Procédé selon la revendication 14, dans lequel ledit échantillon de tissu est un tissu cérébral, du sang ou une fraction sanguine, des globules rouges, des membranes érythrocytaires ou des globules blancs.
